# FASCICULE DE BREVET EUROPEEN

(11) **EP 1 520 560 B1**
(45) Date de publication et mention de la délivrance du brevet: **04.10.2006**
(21) Numéro de dépôt: 04300509.9
(22) Date de dépôt: 03.08.2004
(51) Int. Cl.: A61F 2/40

(54) **Implant huméral pour prothèse de l'épaule**
Humerusimplantat für eine Schulterprothese
Humeral implant for a shoulder prosthesis

(30) Priorité: 01.10.2003 FR 0311501
(43) Date de publication de la demande: 06.04.2005
(73) Titulaire: Biomet France, 26000 Valence (FR); Rio, Bruno, 54000 Nancy (FR); Teissier, Jacques, 34170 Castelnau Le Lez (FR); Toussaint, Bruno, 74940 Annecy le Vieux (FR); Zipoli, Bruno, 40150 Hossegor (FR); Beguin, Laurent, 69540 Irigny (FR); Bouttens, Denis, 62170 St Josse (FR); Cohn, Charles, 83600 Les Adrets de l'Esterel (FR); Declercq, Geert, 2970 Schilde (BE); Grimberg, Jean, 78150 Le Chesnay (FR); Huguet, Dominique, 44700 Orvault (FR); Lesprit, Eric, 33000 Bordeaux (FR); Massart, Pierre, 73100 Tresserve (FR)
(72) Inventeur: Brunnarius, Yann, 07130, Saint Peray (FR); Beguin, Laurent M., 69540 Irigny (FR); Rio, Bruno M., 54000 Nancy (FR); Lesprit, Eric M., 33000 Bordeaux (FR); Massart, Pierre M., 73100 Tresserve (FR); Zipoli, Bruno M., 40150 Hossegor (FR); Teissier, Jacques M., 34170 Castelnau le Lez (FR); Toussaint, Bruno M., 74940 Annecy Le Vieux (FR); Declercq, Geert M., 2970 Schilde (BE); Cohn, Charles M., 83600 Les Adrets de L'Esterel (FR); Bouttens, Denis M., 62170 St. Josse (FR); Grimberg, Jean M., 78150 Le Chesnay (FR); Huguet, Dominique M., 44700 Orvault (FR)
(74) Mandataire: Le Cacheux, Samuel L.R.

(56) Documents cités:
- WO-A-02/39933
- FR-A- 2 674 122
- US-A- 5 725 592

## Description

L'invention concerne le domaine des implants orthopédiques, notamment celui des prothèses d'épaule. De manière générale, ce type de prothèse peut présenter des formes distinctes. Lorsque la cavité glénoïde est intacte, ce qui est notamment le cas dans l'hypothèse d'une fracture comminutive, la prothèse d'épaule comprend un élément huméral fixé dans le canal médullaire de l'humérus, muni d'un implant mâle de forme sphérique, destiné à coopérer directement avec la glène. Un tel dispositif est nommé dans la suite de la description « implant huméral ». Dans le cas d'une destruction de la glène, la prothèse se présente sous la forme d'un support implanté au niveau de la glène et muni d'un insert mâle ou femelle destiné à coopérer avec une forme correspondante agencé au niveau de l'élément huméral.

L'invention concerne plus spécifiquement un implant huméral modulable en ce sens qu'il est conformé pour recevoir, soit un insert mâle, soit un insert femelle, en fonction de la situation rencontrée au niveau de l'articulation de l'épaule considérée. Dans la suite de la description, on désigne par l'expression « insert mâle » une tête prothétique humérale, en pratique une sphère ou demi-sphère, destinée à coopérer avec la cavité glénoïde, soit directement, soit par le biais d'un support muni d'une forme femelle correspondante, en pratique une cupule. De même, l'expression « insert femelle » désigne une partie femelle, en pratique une cupule destinée à coopérer avec une partie mâle, en pratique une sphère ou demi-sphère, agencée sur un support lui même implanté au niveau de la zone glénoïde.

Le document FR-A-2 652 498 décrit un implant huméral du même type comprenant une tige d'ancrage, prolongée dans sa partie supérieure par une tête présentant un évidemment, agencé pour recevoir, d'une manière rapportée, soit un insert mâle, soit un insert femelle. L'un des inconvénients de cette tige est de nécessiter, compte-tenu de l'encombrement de la tête de ladite tige, une résection importante de l'os spongieux. En outre, la mise en place de la tige conduit à modifier le positionnement original de la tête humérale et donc son centre de rotation, risquant d'aboutir à une fracture de la zone métaphysaire de l'humérus.

Le document WO 0 239 933 A représente l'état de la technique le plus proche et détermine le préambule de la revendication 1.

L'objectif de l'invention est donc de proposer un implant huméral modulable qui ne présente pas les inconvénients précités, notamment en terme de résection et d'anatomie.

Dès lors, l'invention a pour objet un implant huméral modulable comprenant une tige d'ancrage et un insert mâle ou un insert femelle aptes à coopérer avec la cavité glénoïde ou un implant glénoïdien. Cet implant se caractérise en ce qu'il comprend en outre un support amovible, destiné à venir s'adapter directement ou indirectement au niveau de l'extrémité supérieure de la tige d'ancrage, et à coopérer avec un col intermédiaire, lui-même muni de moyens de coopération avec l'implant mâle ou femelle.

En d'autres termes, l'invention consiste à avoir prévu, en lieu et place du cône surmontant la tige humérale telle que décrite dans le document FR-A-2 652 498, un support démontable formant panier, conformé pour coopérer, par l'intermédiaire d'un col, avec la tige et recevoir soit un insert mâle, soit un insert femelle.

Pour éviter d'éventuels déplacements des différents éléments les uns par rapport aux autres une fois la prothèse implantée, l'insert male ou femelle de même que l'extrémité supérieure de la tige présentent des moyens de coopération entre eux.

En pratique, le support ou panier se présente sous la forme d'une embase circulaire pourvue d'un orifice traversant, munie d'au moins trois branches, de préférence de quatre branches sensiblement en arc de cercle, identiques et périodiquement réparties, conférant audit support une forme générale hémisphérique. En effet, cette forme hémisphérique est rendue nécessaire par le fait que, comme il sera vu par la suite, tant l'insert mâle que l'insert femelle, également de forme hémisphérique, coopèrent étroitement avec le support. De manière générale tout moyen conférant au support une forme générale hémisphérique peut être envisagé étant rappelé que l'hémisphère coopère avec l'extrémité supérieure de la tige d'ancrage, par son pole et par l'intermédiaire du col, et s'ancre de manière intime et interpénétrante dans l'os spongieux de l'humérus.

Pour permettre le positionnement du support ou panier au niveau de l'extrémité supérieure de la tige, ladite extrémité supérieure présente une succession d'entailles ou de découpes périodiquement réparties, destinées à coopérer avec la base des branches correspondantes du support.

La fixation du support ou panier au niveau de la tige s'effectue au moyen du col précité. Pour éviter tout déplacement du support par rapport à la tige une fois la prothèse implantée, la tige d'ancrage présente, toujours au niveau de son extrémité supérieure, un évidemment de forme cylindrique ou conique, propre à coopérer avec ledit col. En pratique, cet évidemment a un diamètre légèrement inférieur à celui de l'embase du support, de sorte à permettre l'ajustement de celui ci dans la tige.

Dans un mode de réalisation avantageux, l'évidemment de forme cylindrique est muni d'un taraudage propre à coopérer avec un filetage ménagé au niveau de la zone inférieure du col.

Selon une autre caractéristique, la tige présente une section cylindrique, dont le diamètre est quasi constant. En effet, le diamètre est légèrement supérieur au niveau de l'extrémité supérieure par rapport au reste de la tige, la section transversale à ce niveau étant elliptique avec un évasement biconique.

Dans un mode de réalisation avantageux, la tige présente des cannelures en zone distale, permettant d'augmenter les surfaces de contact avec le ciment ou avec l'os, et corollairement de renforcer son ancrage secondaire dans le fut huméral.

Comme déjà dit, l'implant huméral de l'invention est du type modulable en ce sens qu'il peut recevoir un insert mâle, c'est à dire une tête prothétique humérale, ou un insert femelle, c'est à dire une cupule.

Lorsque l'implant reçoit un insert mâle, ledit insert se présente sous la forme d'une hémisphère, dont la base est munie d'un évidement conique, propre à coopérer avec l'extrémité supérieure du col précité. L'autre extrémité du col traverse l'embase du support ou panier, pour venir s'emboîter ou se visser dans l'évidemment supérieur de la tige.

Lorsque l'insert est un insert femelle, ledit insert se présente sous la forme d'une cupule se prolongeant par un axe central positionné à son pole, et faisant fonction du col précité, et destiné à coopérer avec l'évidemment supérieur de la tige. En pratique, le diamètre de la cupule est choisi pour venir s'ajuster dans le support ou panier de forme hémisphérique.

L'invention et les avantages qui en découlent ressortiront mieux des exemples de réalisation qui suivent, donnés à titre indicatif et non limitatif à l'appui des figures annexées.
Les figures 1 et 2 représentent schématiquement l'implant huméral conforme à l'invention, muni d'une tête prothétique humérale, respectivement en vue latérale et en éclaté.
Les figures 3 et 4 représentent schématiquement de manière plus détaillée la partie supérieure de l'implant des figures 1 et 2, et selon une variante de l'invention.
Les figures 5 à 7 représentent schématiquement la tête prothétique humérale conforme à l'invention, respectivement en perspective et vue du dessous.
Les figures 8 et 9 représentent schématiquement l'implant huméral conforme à l'invention, muni d'une cupule, donc d'un insert femelle, respectivement en vue latérale et en éclaté.
La figure 10 est une représentation schématique plus détaillée et en éclaté d'une variante de la forme de réalisation des figures 8 et 9.
La figure 11 est une représentation schématique en perspective du support ou panier conforme à l'invention muni d'une cupule.
Les figures 12 et 13 sont des représentations schématiques en perspective de la cupule de l'invention, respectivement vue du dessus et du dessous.
La figure 14 est une représentation schématique en perspective de l'insert en polyéthylène venant s'enclipser dans la cupule des figures 12 et 13.

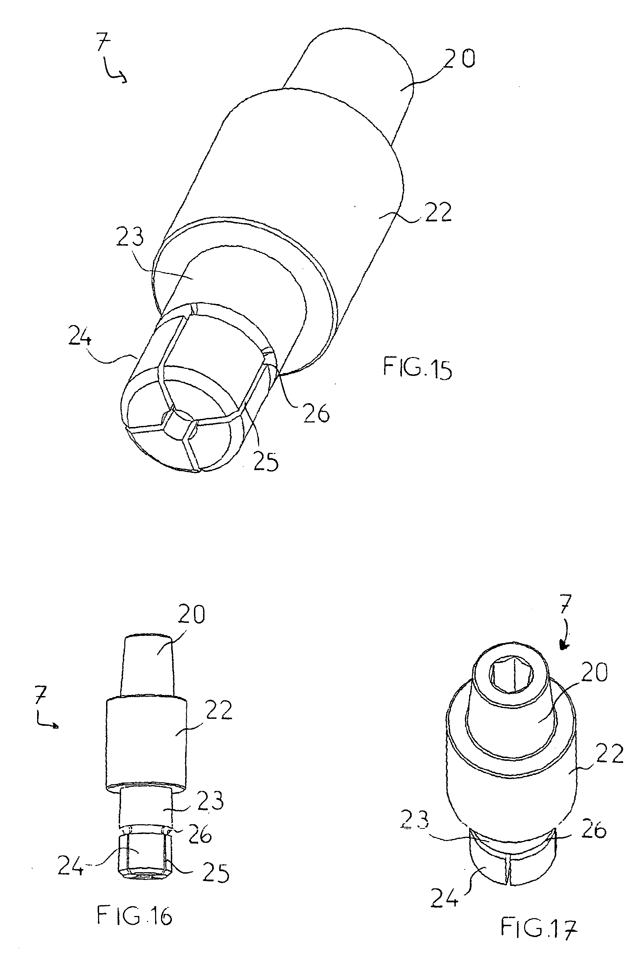

Les figures 15 à 17 sont des représentations schématiques en perspective du col destiné à coopérer avec la tige de l'implant de l'invention.
Les figures 18 et 19 sont des représentations schématiques en perspective du support ou panier conforme à l'invention.
Les figures 20 et 21 sont des représentations schématiques en perspective d'un élément additionnel susceptible d'être mis en oeuvre dans le cadre de la présente invention.
Les figures 22 et 23 sont des représentations schématiques en perspective de la tige conforme à l'invention, selon deux vues différentes.

Selon l'invention, l'implant huméral est constitué de trois éléments distincts, respectivement une tige d'ancrage (1) ou (8), un support (5) et un insert mâle ou un insert femelle.

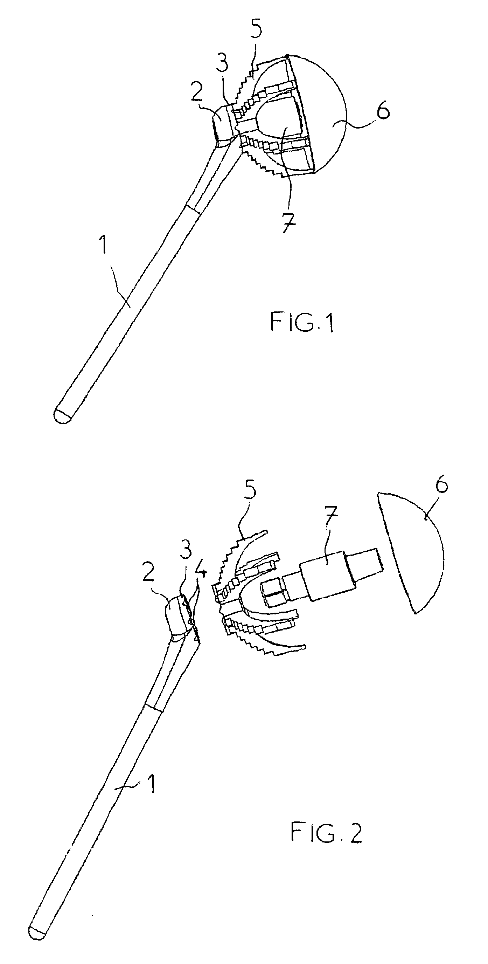

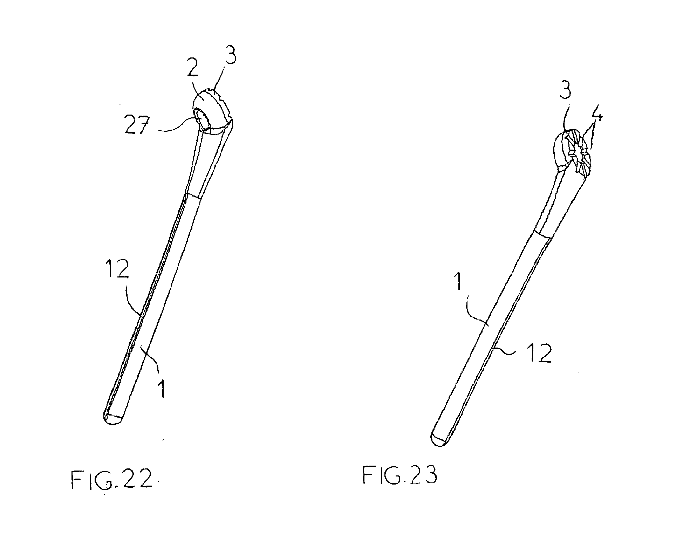

Comme représenté sur les figures 1 et 2, et par ailleurs sur les figures 22 et 23, la tige d'ancrage (1) a une forme générale cylindrique de section quasi constante. Cependant, son diamètre est légèrement supérieur au niveau de l'extrémité supérieure par rapport au reste de la tige, la section transversale à ce niveau étant elliptique avec un évasement biconique.

En outre, sa partie distale présente des cannelures (12) propres à optimiser son ancrage secondaire au sein de l'os huméral.

Selon l'invention, la tige (1) est destinée à recevoir un support en forme de panier (5), lui-même destiné à recevoir un insert mâle ou femelle.

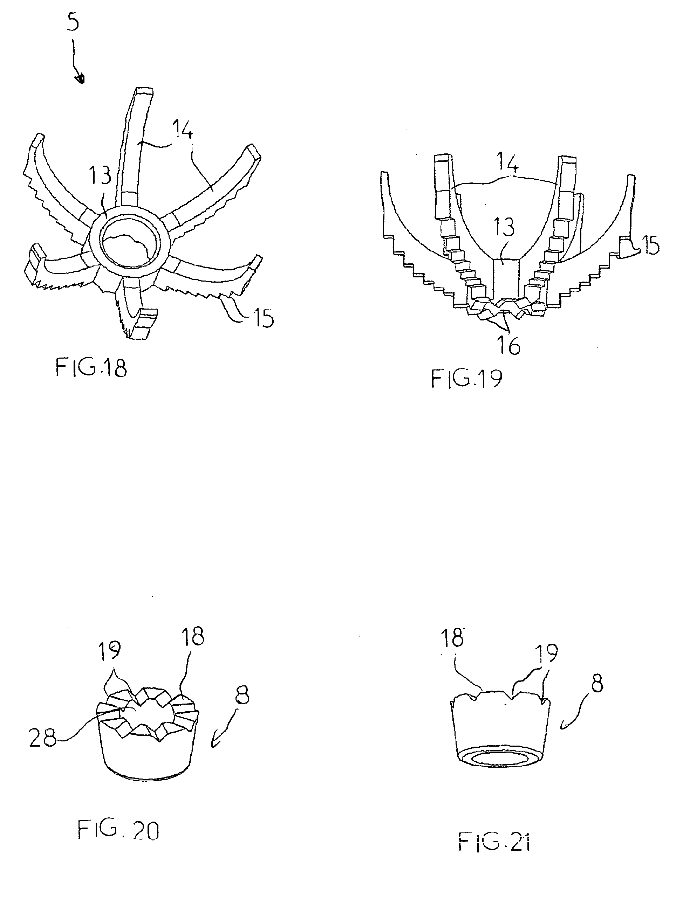

Ce panier (5), que l'on peut mieux visualiser sur les figures 18 et 19, est constitué d'une embase cylindrique (13), de laquelle s'étendent une pluralité de branches (14), identiques, périodiquement espacées les unes des autres. Ces branches en forme générale d'arc de cercle, définissent une enveloppe sensiblement hémisphérique. La face externe des branches est munie de dents, destinées à favoriser l'ancrage dudit support dans l'os spongieux de l'humérus. En outre, l'embase cylindrique (13) est tout d'abord percée d'un alésage (17), destiné à permettre le passage d'un col (7), décrit plus en détail ultérieurement, et présente en outre sur sa face inférieure des dentures radiales (16), périodiquement réparties, et destinées à permettre le positionnement correct du panier (5) sur l'extrémité supérieure (3) de la tige d'ancrage (1) ou sur la face supérieure (18) d'un élément intermédiaire ou tige courte (8), décrit en relation avec les figures 20 et 21.

De fait, tant la face supérieure (18) de l'élément intermédiaire (8) que l'extrémité supérieure (3) de la tige sont munies de nervures (19, 4) de forme et de pas correspondants, propres à coopérer avec ces dentures radiales (16).

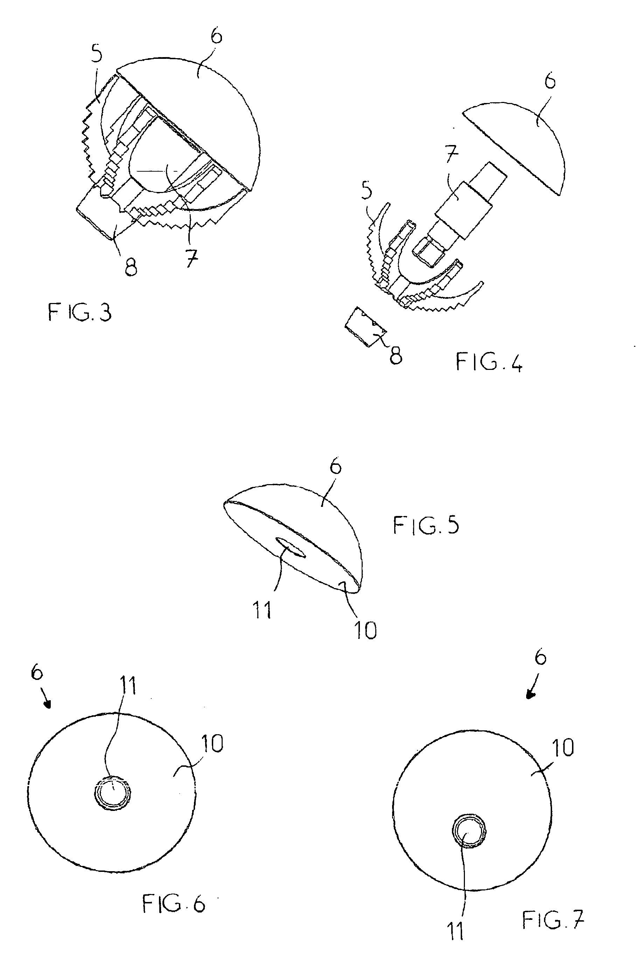

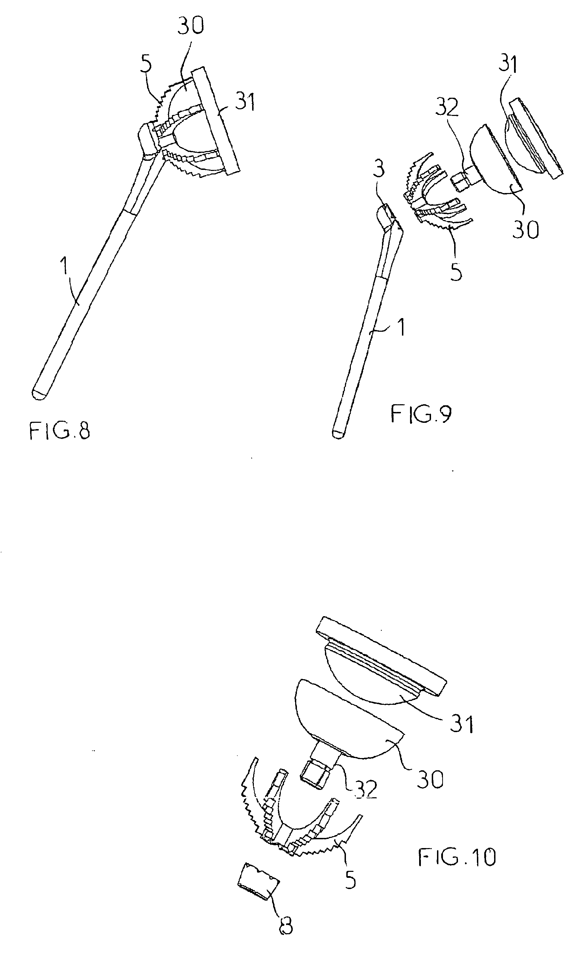

L'insert mâle ou tête prothétique humérale (6) est constitué par une calotte sensiblement hémisphérique, notamment réalisée en acier inoxydable, en alliage chrome-cobalt, ou en tout autre matériau. La base (10) de cette calotte est pourvue d'un alésage (11) centré (figure 6) ou excentré (figure 7), en fonction de l'orientation souhaitée par le praticien. Cet alésage est destiné à recevoir l'extrémité supérieure (20) du col (7). Celle-ci se présente sous la forme d'un cône morse, propre à venir s'emmancher au sein de l'alésage (10), de forme correspondante. Il peut être observé que ladite extrémité supérieure (20) est également pourvu d'un alésage (21), de section polygonale, afin de permettre l'introduction d'un outil de pose dudit col (7) sur l'extrémité supérieure (3) de la tige d'ancrage (1). Cette extrémité supérieure (20) se prolonge de manière colinéaire par une portion (22) de diamètre plus important, sur laquelle repose ledit insert mâle (6). Cette portion (22) se prolonge à son tour, également de manière colinéaire par une zone cylindrique (23), puis par une extrémité inférieure (24), présentant soit une forme de cône morse, soit un filetage (25), ces deux zones étant séparées par une zone (26) de section plus réduite. Cette zone (26) est destinée à conférer une certaine souplesse à l'extrémité inférieure (24).

La zone cylindrique (23) est destinée à coopérer avec l'alésage (17) de l'embase (13) du panier (5), et s'étend de fait sur une longueur correspondant sensiblement à la hauteur de ladite embase, et présente un diamètre correspondant au jeu près au diamètre interne dudit alésage.

L'extrémité inférieure (24) est quant à elle destinée à coopérer avec un évidement (27), ménagé au sein de l'extrémité supérieure (3) de la tige (1), ou avec un évidement (28), ménagé au sein de l'élément intermédiaire ou tige courte (8), afin d'assurer la fixation dudit col auprès de ces deux éléments respectifs, et corollairement la fixation du panier (5) et de l'implant (6) sur la tige d'ancrage (1). Ces évidements (27, 28) sont de fait soit pourvu d'une forme propre à coopérer avec la forme en cône morse de ladite extrémité inférieure (24), soit pourvu d'un taraudage de pas correspondant au filetage (25), dont est le cas échéant pourvue ladite extrémité inférieure (24).

Dans le cas de l'insert mâle, le panier (5) assure une stabilisation de la tige, et favorise en outre l'ancrage de l'implant au sein de l'humérus.

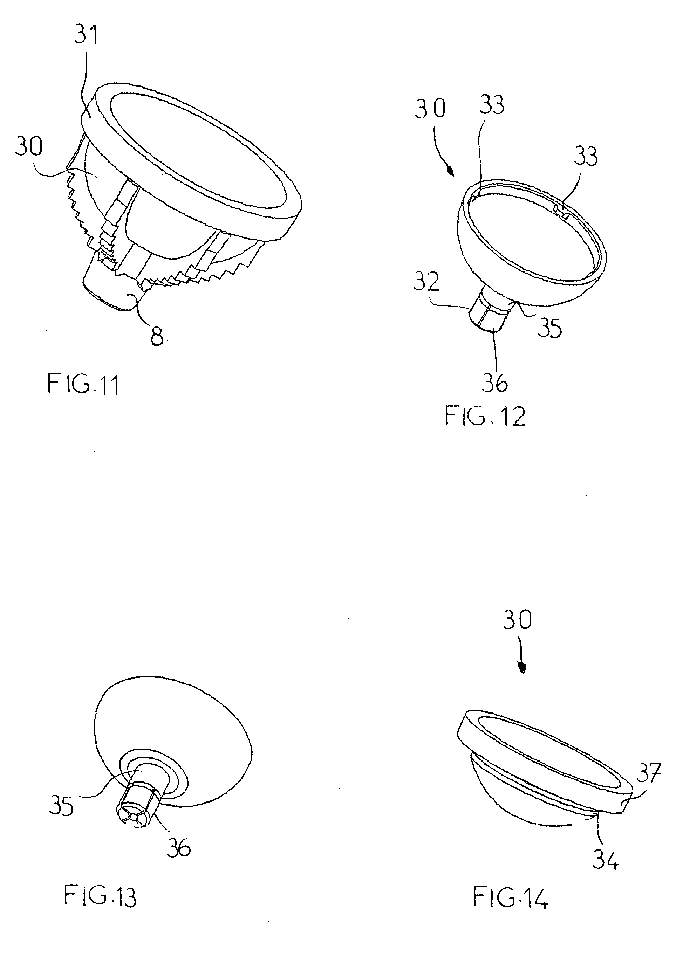

L'insert femelle, plus particulièrement représenté en relation avec les figures 11 à 14, est constitué d'une cupule (30), au sein de laquelle vient s'enclipser un insert en polyéthylène (31).

La cupule (30), de forme sensiblement hémisphérique, se prolonge par un axe (32) à partir de son pôle inférieur, ledit axe (32) remplissant la même fonction que le col (7) précité, et est donc muni comme ce dernier :
- d'une zone intermédiaire cylindrique (35), destinée à coopérer avec l'alésage (17) de l'embase (13) du panier (5),
- et d'une zone inférieure (36), destinée à coopérer avec l'évidement (27) de l'extrémité supérieure (3) de la tige d'ancrage (1), ou (28) de l'élément intermédiaire (8).

L'enveloppe externe de la cupule (30) correspond sensiblement au volume interne défini par le panier (5).

L'insert polyéthylène (31) vient s'enclipser au sein de la cupule (30). Pour ce faire, il est muni de nervures annulaires (34), venant coopérer avec des ergots (33) formant saillie en direction du volume défini par la cupule, et ménagés au voisinage du rebord supérieur de ladite cupule. La périphérie de l'insert (31) est en outre munie d'une collerette (37), destinée à venir prendre appui sur la cupule (30).

Ainsi qu'on l'aura compris, l'invention comporte deux modes principaux de réalisation. L'un met en oeuvre une tige longue (1), destinée à être insérée dans le canal médullaire de l'humérus. L'autre met en oeuvre une tige courte (8), lorsque l'implantation d'une tige longue est superflue. Cette tige courte permet ainsi de réaliser l'implantation directe de l'implant huméral sur l'humérus.

Comme déjà dit, l'un des avantages de cet implant est qu'il nécessite une résection limitée de l'os spongieux. La mise en place de l'implant est effectuée au moyen d'instruments en forme de croix qui viennent épouser la forme du support en panier, ce qui limite les pertes osseuses.

Une fois en place, l'insert mâle ou femelle ne bouge plus grâce à la coopération étroite entre les différents éléments.

L'invention et les avantages qui en découlent ressortent bien de la description qui précède, on note notamment la forme anatomique de la tige d'ancrage, de même que les propriétés modulaires de l'implant.

## Revendications

1. Implant huméral modulable comprenant :
• une tige d'ancrage (1, 8) ;
• un insert mâle (6) ou un insert femelle (30, 31), aptes à coopérer avec la cavité glénoïde ou un implant glénoïdien,
• un support amovible (5), destiné à venir s'adapter directement ou indirectement au niveau de l'extrémité supérieure (3) de la tige d'ancrage (1, 8), et à coopérer avec un col intermédiaire (7, 32), lui-même muni de moyens de coopération avec l'implant mâle ou femelle ;
***caractérisé* en ce que** le support amovible (5) est en forme de panier muni d'une embase circulaire (13) pourvue d'un orifice traversant (17), et comportant au moins trois branches (14), sensiblement en arc de cercle, identiques et périodiquement réparties, conférant audit support une forme générale hémisphérique.

2. Implant huméral modulable selon la revendication 1, ***caractérisé* en ce que** la face externe des branches (14) est munie de dents (15), destinées à coopérer avec l'os spongieux.

3. Implant huméral modulable selon l'une des revendications 1 et 2, ***caractérisé* en ce que** l'embase cylindrique (13) du support (5) présente sur sa face inférieure des dentures radiales (16), périodiquement réparties, et destinées à permettre le positionnement correct du panier (5) sur l'extrémité supérieure (3, 18) de la tige d'ancrage (1, 8).

4. Implant huméral modulable selon l'une des revendications 1 à 3, ***caractérisé* en ce que** la tige d'ancrage (1, 8) présente, au niveau de son extrémité supérieure (3), un évidement (27, 28) de forme tronconique, destiné à coopérer avec l'extrémité inférieure (24) également de forme tronconique du col (7).

5. Implant huméral modulable selon l'une des revendications 1 à 3, ***caractérisé* en ce que** la tige d'ancrage (1, 8) présente, au niveau de son extrémité supérieure (3), un évidement (27, 28) de forme cylindrique, muni d'un taraudage, destiné à coopérer avec l'extrémité inférieure (24) du col (7) pourvue d'un filetage (25) correspondant.

6. Implant huméral modulable selon l'une des revendications 1 à 5, ***caractérisé* en ce que** le col intermédiaire (7) présente trois segments colinéaires :
- l'extrémité supérieure (20) se présentant sous la forme d'un cône morse, propre à coopérer avec l'insert mâle (6) ;
- une portion (22) de diamètre plus important, sur laquelle repose ledit insert mâle (6) ;
- une zone cylindrique (23), destinée à coopérer avec l'alésage (17) de l'embase (13) du panier (5), et se prolongeant par une extrémité inférieure (24), présentant soit une forme de cône morse, soit un filetage (25), ces deux zones étant séparées par une zone (26) de section plus réduite.

7. Implant huméral modulable selon la revendication 6, ***caractérisé* en ce que** l'insert mâle ou tête prothétique huméral (6) est constitué d'une hémisphère, dont la base (10) est munie d'un évidement conique (11), centré ou excentré, propre à coopérer avec l'extrémité supérieure (20) du col (7).

8. Implant huméral modulable selon la revendication 6, ***caractérisé* en ce que** l'insert femelle (30, 31) est constitué par une cupule (30), dont l'enveloppe externe correspond sensiblement au volume interne défini par le panier (5), et se prolongeant par un axe central (32) positionné à son pole, et remplissant la même fonction que le col (7), et destiné à coopérer avec l'évidement (17) de la tige (1), ladite cupule (30) étant destinée à recevoir un insert en polyéthylène (31), qui vient s'enclipser au sein de la cupule (30).

## Claims

1. A modular humeral implant comprising:
• an anchoring stem (1, 8);
• a male insert (6) or a female insert (30, 31), able to cooperate with the glenoid cavity or a glenoidal implant,
• a removable support (5), intended to be directly or indirectly adapted at the upper end (3) of the anchoring stem (1, 8), and to cooperate with an intermediate neck (7, 32) itself provided with means for cooperating with the male or female implants;
**characterized in that** the removable support (5) has the shape of a basket provided with a circular base (13) provided with a through-hole (17) and including at least three identical and periodically distributed branches (14) substantially as a circular arc, giving said support a general hemispherical shape.

2. The modular humeral implant according to claim 1, **characterized in that** the external face of the branches (14) is provided with teeth (15), intended to cooperate with the spongious bone.

3. The modular humeral implant according to any of claims 1 and 2, **characterized in that** the cylindrical base (13) of the support (5) has on its lower face radial teeth (16) periodically distributed and intended to allow the basket (5) to be properly positioned on the upper end (3, 18) of the anchoring stem (1, 8).

4. The modular humeral implant according to any of claims 1 to 3, **characterized in that** the anchoring stem (1, 8) has at its upper end (3), a recess (27, 28) with a frustro-conical shape, intended to cooperate with the lower end (24) also with a frustro-conical shape, of the neck (7).

5. The modular humeral implant according to any of claims 1 to 3, **characterized in that** the anchoring stem (1, 8) has at its upper end (3), a recess (27, 28) with a cylindrical shape, provided with a female thread, intended to cooperate with the lower end (24) of the neck (7) provided with a matching male thread (25).

6. The modular humeral implant according to any of claims 1 to 5, **characterized in that** the intermediate neck (7) has three collinear segments;
- the upper end (20) appearing as a Morse taper, able to cooperate with the male insert (6);
- a portion (22) with a larger diameter, on which said male insert (6) rests;
- a cylindrical area (23), intended to cooperate with the bore (17) of the base (13) of the basket (5), and extending with a lower end (24), having either a Morse taper shape or a thread (25), both of these areas being separated by an area (26) with a more reduced section.

7. The modular humeral implant according to claim 6, **characterized in that** the male insert or humeral prosthetic head (6) consists of a hemisphere, the base (10) of which is provided with a conical recess (11), either centred or eccentric, able to cooperate with the upper end (20) of the neck (7).

8. The modular humeral implant according to claim 6, **characterized in that** the female insert (30, 31) is formed with a cup (30), the external envelope of which substantially corresponds to the internal space defined by the basket (5), and extending with a central axis (32) positioned at its pole, and fulfilling the same function as the neck (7), and intended to cooperate with the recess (17) of the stem (1), said cup (30) being intended to receive a polyethylene insert (31), which will be attached with clips within the cup (30).

## Patentansprüche

1. Variierbares Humerusimplantat umfassend:
• einen Verankerungsschaft (1, 8);
• einen Steckeinsatz (6) oder einen aufnehmenden Einsatz (30, 31), die geeignet sind, mit der Gelenkpfanne oder einem Glenoidimplantat zusammenzuwirken,
• einen lösbaren Träger (5), welcher dazu bestimmt ist, sich direkt oder indirekt im Bereich des oberen Endes (3) des Verankerungsschaftes (1, 8) einzupassen und mit einem Zwischenhals (7, 32) zusammenzuwirken, der selbst mit Mitteln zum Zusammenwirken mit dem Steck- oder aufnehmenden Implantat versehen ist;
**dadurch gekennzeichnet, daß** der lösbare Träger (5) die Form eines Korbes aufweist, der mit einem kreisförmigen Sockel (13) ausgestattet ist, welcher mit einer durchgehenden Öffnung (17) versehen ist und wenigstens drei im wesentlichen kreisbogenförmige, identische und in regelmäßigem Abstand verteilte Schenkel (14) aufweist, wodurch dem Träger eine allgemeine Halbkugelform verliehen wird.

2. Variierbares Humerusimplantat nach Anspruch 1, **dadurch gekennzeichnet, daß** die Außenseite der Schenkel (14) mit Zähnen (15) versehen ist, die dazu bestimmt sind, mit dem spongiösen Knochen zusammenzuwirken.

3. Variierbares Humerusimplantat nach einem der Ansprüche 1 und 2, **dadurch gekennzeichnet, daß** der zylinderförmige Sockel (13) des Trägers (5) an seiner Unterseite Radialverzahnungen (16) aufweist, die in regelmäßigem Abstand verteilt und dazu bestimmt sind, die korrekte Positionierung des Korbes (5) auf dem oberen Ende (3, 18) des Verankerungsschaftes (1, 8) zu ermöglichen.

4. Variierbares Humerusimplantat nach einem der Ansprüche 1 bis 3, **dadurch gekennzeichnet, daß** der Verankerungsschaft (1, 8) im Bereich seines oberen Endes (3) eine kegelstumpfförmige Aussparung (27, 28) aufweist, die dazu bestimmt ist, mit dem ebenfalls kegelstumpfförmigen unteren Ende (24) des Halses (7) zusammenzuwirken.

5. Variierbares Humerusimplantat nach einem der Ansprüche 1 bis 3, **dadurch gekennzeichnet, daß** der Verankerungsschaft (1, 8) im Bereich seines oberen Endes (3) eine zylinderförmige, mit einem Innengewinde versehene Aussparung (27, 28) aufweist, die dazu bestimmt ist, mit dem unteren Ende (24) des Halses (7), das mit einem entsprechenden Gewinde (25) versehen ist, zusammenzuwirken.

6. Variierbares Humerusimplantat nach einem der Ansprüche 1 bis 5, **dadurch gekennzeichnet, daß** der Zwischenhals (7) drei kollineare Abschnitte aufweist:
- das obere Ende (20), das in Form eines Morsekegels vorliegt und das geeignet ist, mit dem Steckeinsatz (6) zusammenzuwirken;
- einen Abschnitt (22) größeren Durchmessers, auf dem der Steckeinsatz (6) ruht;
- einen zylindrischen Bereich (23), der dazu bestimmt ist, mit der Bohrung (17) des Sockels (13) des Korbes (5) zusammenzuwirken und der sich durch ein unteres Ende (24) fortsetzt, das entweder die Form eines Morsekegels oder ein Gewinde (25) aufweist, wobei diese beiden Bereiche durch einen Bereich (26) kleineren Querschnitts getrennt sind.

7. Variierbares Humerusimplantat nach Anspruch 6, **dadurch gekennzeichnet, daß** der Steckeinsatz oder prothetische Humeruskopf (6) aus einer Halbkugel besteht, deren Basis (10) mit einer zentrierten oder exzentrierten kegelförmigen Aussparung (11) versehen ist, die geeignet ist, mit dem oberen Ende (20) des Halses (7) zusammenzuwirken.

8. Variierbares Humerusimplantat nach Anspruch 6, **dadurch gekennzeichnet, daß** der aufnehmende Einsatz (30, 31) von einem Becher (30) gebildet ist, dessen Außenhülle im wesentlichen dem durch den Korb (5) definierten Innenvolumen entspricht und der durch eine an seinem Pol angeordnete Mittelachse (32) verlängert ist, welche dieselbe Funktion wie der Hals (7) erfüllt und dazu bestimmt ist, mit der Aussparung (17) des Schaftes (1) zusammenzuwirken, wobei der Becher (30) dazu bestimmt ist, einen Polyethyleneinsatz (31) aufzunehmen, der innerhalb des Bechers (30) eingeclipst wird.
